Europäisches Patentamt

(19) European Patent Office

Office européen des brevets

(11) Publication number: **0 079 738**
**B1**

(12) **EUROPEAN PATENT SPECIFICATION**

(45) Date of publication of patent specification: **10.06.87**

(51) Int. Cl.⁴: **H 01 J 29/07**

(21) Application number: **82305925.8**

(22) Date of filing: **08.11.82**

(54) **Metal component for a colour cathode ray tube.**

(30) Priority: **09.11.81 JP 178371/81**

(43) Date of publication of application:
**25.05.83 Bulletin 83/21**

(45) Publication of the grant of the patent:
**10.06.87 Bulletin 87/24**

(84) Designated Contracting States:
**DE FR GB**

(56) References cited:
**DE-A-2 945 467**
**FR-A-2 231 101**
**US-A-3 647 572**
**US-A-3 838 985**
**US-A-4 292 565**

(73) Proprietor: **Kabushiki Kaisha Toshiba**
**72, Horikawa-cho Saiwai-ku**
**Kawasaki-shi Kanagawa-ken 210 (JP)**

(72) Inventor: **Kanto, Masaharu c/o Patent Division**
**Tokyo Shibaura Denki K.K. 72, Horikawa-cho**
**Saiwai-ku Kawasaki-shi Kanagawa-ken (JP)**
Inventor: **Kihara, Hisato c/o Patent Division**
**Tokyo Shibaura Denki K.K. 72, Horikawa-cho**
**Saiwai-ku Kawasaki-shi Kanagawa-ken (JP)**
Inventor: **Teshima, Koichi c/o Patent Division**
**Tokyo Shibaura Denki K.K. 72, Horikawa-cho**
**Saiwai-ku Kawasaki-shi Kanagawa-ken (JP)**
Inventor: **Inaba, Michihiko c/o Patent Division**
**Tokyo Shibaura Denki K.K. 72, Horikawa-cho**
**Saiwai-ku Kawasaki-shi Kanagawa-ken (JP)**

(74) Representative: **Kirk, Geoffrey Thomas et al**
**BATCHELLOR, KIRK & EYLES 2 Pear Tree Court**
**Farringdon Road**
**London EC1R 0DS (GB)**

Courier Press, Leamington Spa, England.

0 079 738

**Description**

This invention relates to metal components which are suitable for use in a colour cathode ray tube. In particular, the invention relates to the shadow mask, the frame for supporting the shadow mask and the beam shield arranged for attachment to the frame which are components employed in a colour cathode ray tube.

Generally, these components are formed from a metal which is predominantly iron and, for various reasons, the surfaces of the components are oxidised. The main reasons are to prevent unwanted and uncontrolled rusting of the components before they are fitted into the tube, to increase the efficiency of heat radiation, and also reduce the reflection of the electron beam when the cathode ray tube, in which the components are fitted, is in use (see e.g. US—A—4292565).

The oxidised layers are formed by heat treatment of the components in a controlled atmosphere containing oxygen, after the components have been formed by a pressing operation. The oxidised layer is generally a bluish black colour and, as a result, the layer is sometimes referred to as a blackened layer.

However, the oxidised layers on the components do cause problems in that they are generally weak in adhesion to the metal components and they easily peel off the components. Particles which have peeled off can seriously affect the operation of the tube by lodging in some of the apertures of the shadow mask or by causing an electrical discharge to occur between electrodes in the tube.

It is an object of the present invention to improve the adhesion of the oxidised layer on a metal component suitable for use in a colour cathode ray tube.

According to the present invention, a metal component of a colour cathode ray tube is formed mainly of iron and has an oxidised layer on its surface, characterised in that the metal includes silicon (Si), aluminium (Al) and chromium (Cr), the proportions by weight of these elements satisfy the equation

$$Cr \geq 1/3 \ (Al+Si),$$

and the combined amount of Al, Si and Cr being equal to or less than 0.44% by weight, thereby improving the adhesion of the oxidised layer to said component.

Preferably, the proportions in weight satisfy the equation:—

$$Cr \geq (Al+Si).$$

The component may be in the form of a shadow mask, or a mask frame, or a shield for attachment to the frame.

In order that the invention may be more readily understood, it will now be described, by way of example only, with reference to the accompanying drawings, in which:—

Figure 1 is a cross-section of a colour cathode ray tube;

Figure 2 is a flow chart for manufacturing the shadow mask fitted in the tube;

Figure 3 is a microscopic photograph showing the oxidised layer after an adhesion test;

Figure 4 is a microscopic photograph showing another oxidised layer after the adhesion test;

Figure 5 is a chart showing the contained amounts of the elements along the depth direction of the shadow mask;

Figure 6 is a chart showing the contained amounts of the elements along the depth direction of another shadow mask; and

Figure 7 is a chart showing a relationship between the contained amounts of chromium and (aluminium and silicon).

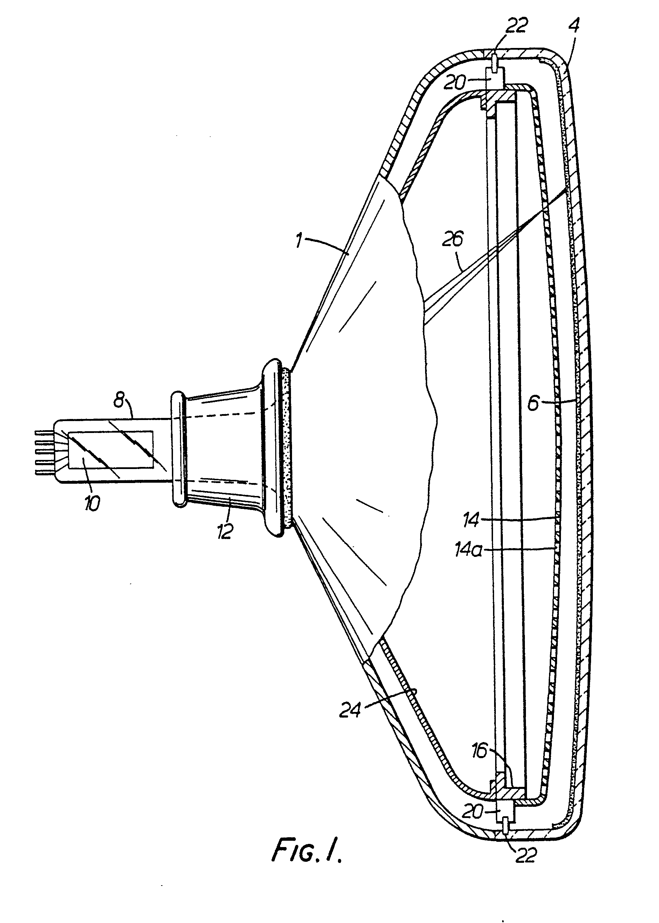

Referring now to Figure 1, there is shown a cross-section of a colour cathode ray tube. A funnel 2 is joined to the outer periphery of a face plate 4, on the inner surface of which is formed a phosphor screen 6. A neck 8 is joined to an end of funnel 2. An electron gun 10 is disposed within neck 8. A deflection yoke 12 is mounted on the outer surface of funnel 2 and around neck 8. A shadow mask 14, having a plurality of apertures 14a is attached to a mask frame 16 having resilient support members 20. Support members 2 are demountably attached to pins 22 embedded in the face plate 4 and arranged so that shadow mask 14 faces the phosphor screen on inner surface of face plate 4. An inner shield 24, which is provided for shielding electron beams 26 from the earth's magnet field, is attached to the mask frame 16 and extends along the funnel.

Electron beams 26 emitted from the electron gun are deflected by the deflection yoke 12, pass through an aperture of shadow mask 14 and impinge upon the phosphor screen 6 to cause a colour image to appear on the face of the tube.

In the above-described structure, one or more of the shadow mask 14, mask frame 16 and inner shield 24 are made of metal containing iron as its chief element and including small amounts of silicon (Si), aluminium (Al) and chromium (Cr).

The surface layers are oxidised.

Experiments conducted by the inventors in connection with the development of the present invention will be described below.

The inventors made a shadow mask from a raw steel material that is easily precisely etched and is not

2

# 0 079 738

very susceptible to stretcher strain during press forming. The raw steel material was prepared by a continuous casting process including the addition of aluminium and including a decarbonising anneal. The use of a continuous casting process decreases impurities and non-metal mixtures in the raw steel material to a high degree and the uniformity of the raw steel material is improved. The preciseness of etching is thereby improved. Carbon and nitrogen increase the yield point and stretcher with yield point, and they degrade the press forming performance. Consequently, aluminium is added into the raw steel material to fix nitrogen as aluminium nitride and the decarbonising anneal is performed to reduce carbon, and thereby stretcher with yield point is suppressed and an occurrence of stretcher strain is prevented.

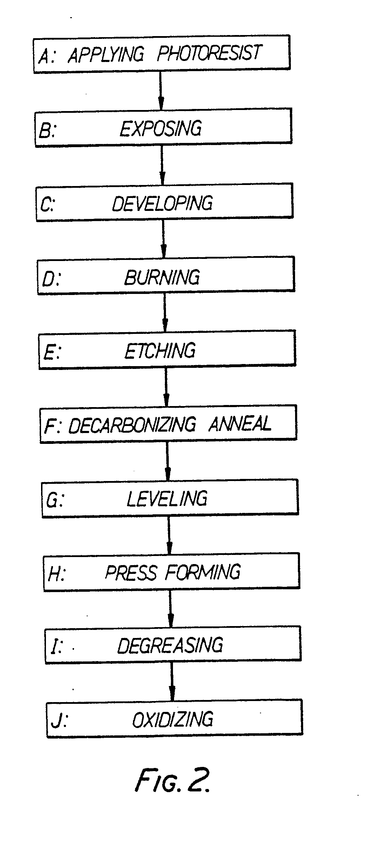

Referring now to Figure 2, the steps of manufacture of the shadow mask from raw steel material are set forth. Step A represents the coating of a photoresist material on the steel strip. Step B represents exposing the photoresist material with a dots or slits pattern of light. In step C, the exposed photoresist material is developed. In step D, the developed photoresist material is heated to harden it. This step is sometimes referred to as burning step. Step E represents etching of the steel strip with the developed photoresist material thereon. The etching of the steel strip creates apertures in it. After etching in step E, washing, drying and cutting steps are performed (not represented in the block diagram). The cutting provides a plurality of flat masks for colour cathode ray tubes. After cutting, and in step F, a flat mask is annealed at a temperature of about 700°C in order to decarbonise it. After decarbonising, a levelling operation is carried out at step G, in which step the flat mask is rolled for flatness adjustment. At step H, press forming is carried out to provide the apertured flat mask with a curved shape. Step I represents a degreasing of the curved mask. In oxidising step J, the shadow mask is oxidised by heat treatment at a temperature of about 600°C in an atmosphere of $CO_2$—$N_2$ including a very small amount of $H_2O$ or $O_2$.

The used raw steel material contains so much aluminium, which is added for fixing nitrogen, that the oxidised layer formed on the surface of the shadow mask tends to be easily peeled off. The inventors conducted several experiments and they found that the adhesion of the oxidised layer depends on not only the amount of aluminium in the shadow mask but also the amount of chromium. The experimental data is shown in Table 1.

TABLE 1

| Sample | C | Composition (ppm) | | | | | | Adhesion |
|--------|---|-----|-----|-----|-----|-----|-----|----------|
| | | Mn | S | P | Si | Al | Cr | |
| (a) | 55 | 2350 | 130 | 161 | 10 | 1 | 489 | ○ |
| (b) | 36 | 2870 | 228 | 157 | 40 | 479 | 182 | Δ |
| (c) | 39 | 2660 | 191 | 153 | 130 | 397 | 74 | × |

○: good;
Δ: good, however partially peeled off; and
×: bad.

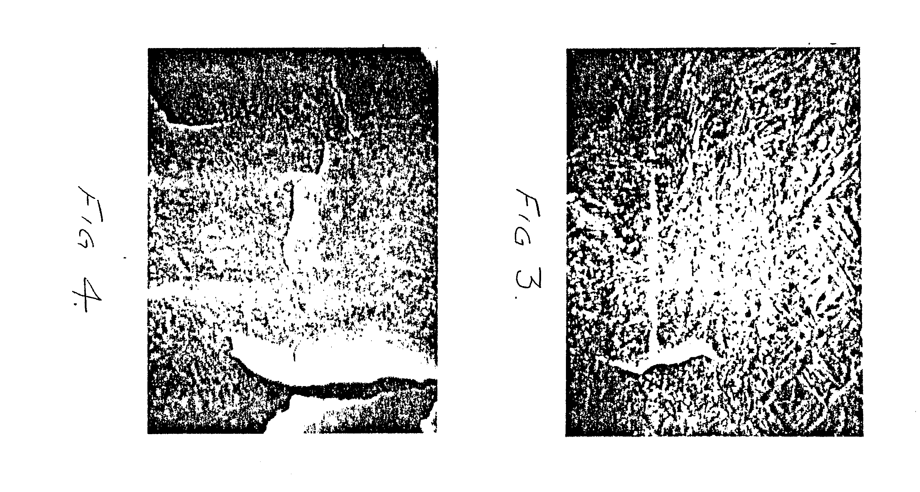

The test was conducted as follows. The oxidised shadow mask was bent at an angle of 90° and an adhesive cellophane tape was applied to the bent portion and then removed. The result of the test showed that sample (a) demonstrated good adhesion without any peeling, as shown in Figure 3. On the other hand, sample (c) demonstrated an adhesion, as shown in Figure 4, which indicated cracks of the oxidised layer.

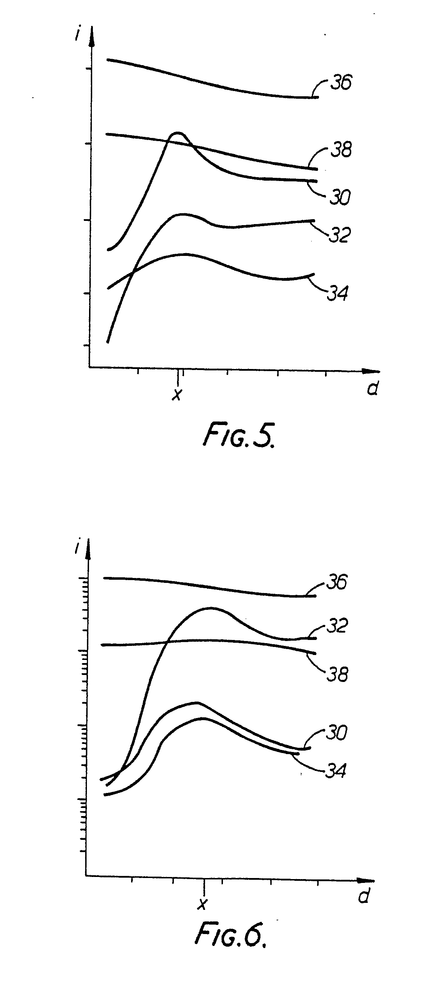

Further, the inventors conducted analysis of the composition along the depth direction from the surface of the shadow mask by an ion micro analyzer. Figures 5 and 6 show the test results. The horizontal axes indicate depth (d) from the surface of the shadow mask and the vertical axes indicate the detection intensity on a logarithmic scale. An x-mark on each horizontal axis denotes the border between the oxidised layer and the shadow mask. Figure 5 shows the test results for sample (a) and Figure 6 shows the test results for sample (c). Curves for chromium, aluminium and silicon 30, 32 and 34, respectively, gradually increase from the surface of oxidised layer to the border, and have maximum values near border x. Behind the border, that is in the shadow mask, they are relatively constant with changing depth. On the contrary, the curves for iron and manganese 36 and 38, respectively, show relatively constant values through their entire cross section of both the oxidised layer and the shadow mask. A typical difference between Figures 5 and 6 is found in a relation between curves of chromium and aluminium 30 and 32, respectively. Curves of chromium, aluminium and silicon 30, 32 and 34, respectively, are extremely different from curves of iron and manganese 36 and 38. This demonstrates that chromium, aluminium and silicon affect the adhesion of the oxidised layer. Therefore, the inventors conducted a further test wherein the amounts of chromium, aluminium and silicon were changed. This further test is reported in Table 2 and was carried out in a similar fashion to the test reported in Table 1.

3

TABLE 2

| Sample | Cr | Al | Si | Unit (ppm) Al+Si | Adhesion |
|---|---|---|---|---|---|
| (a) | 489 | 1 | 10 | 11 | ○ |
| (d) | 980 | 364 | 231 | 595 | ○ |
| (e) | 2014 | 895 | 207 | 1192 | ○ |
| (f) | 2390 | 1932 | 78 | 2010 | ○ |
| (g) | 2005 | 350 | 1200 | 1550 | ○ |
| (b) | 182 | 479 | 40 | 539 | Δ |
| (h) | 780 | 1323 | 157 | 1480 | Δ |
| (i) | 1620 | 1895 | 275 | 2170 | Δ |
| (j) | 993 | 1233 | 1340 | 2573 | Δ |
| (c) | 74 | 397 | 130 | 527 | × |
| (k) | 195 | 562 | 748 | 1310 | × |
| (l) | 410 | 1237 | 778 | 2105 | × |
| (m) | 576 | 1250 | 1330 | 2580 | × |

○: good,
Δ: good, however partially peeled off; and
×: bad.

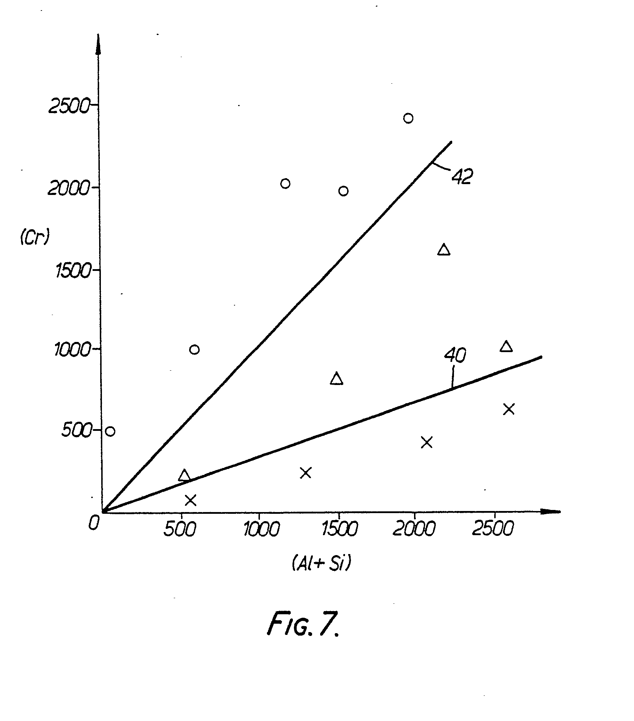

It is difficult to find a relation between chromium, aluminium and silicon individually with respect to the adhesion of the oxidised layer. However, it can be found that a mutual relation exists between chromium and (aluminium+silicon). Figure 7 shows this mutual relation. The horizontal axis indicates an amount of (aluminium+silicon) and the vertical axis indicates an amount of chromium. The ○ marks denote good and their × marks denote bad adhesion qualities. The Δ marks denote good, however, partially peeling off are recognised. Improvement of adhesion is found in the region above a straight line 40 representing Cr=1/3 (Al+Si), as shown in Figure 7. The region above a straight line 42, which is indicated by Cr=(Al+Si), is particularly preferable.

With respect to the amount of silicon, which is added as a deoxidising agent during manufacturing the raw steel material and inevitably remains therein, it is found that an increase in amount of silicon does not harden the raw steel material more than necessary under the above-mentioned condition, even though conventionally it causes a phenomenon of hardening the raw steel material. This is confirmed by example (g), shown in Table 2. It can be considered that chromium fixes carbon as chromium carbide and the ease in forming the shadow mask is thereby improved.

The present invention is accomplished from the above-described experiments and the discussions. Therefore, according to the present invention, a component for use in the colour cathode ray tube has an oxidised layer on its surface and is formed from a metal which includes iron as its chief element and includes chromium, aluminium and silicon. The weight ppm amounts of chromium, aluminium and silicon is sufficient to improve the adhesion of the oxidised layer to the component and meets the following requirement:—

$$Cr \geq 1/3 \ (Al+Si).$$

The raw materials are less susceptible to stretcher strain and are easily precisely attached. The raw steel material can be made by a continuous casting process to reduce impurities and non-metal impurities for improving the precision of etching. Further, nitrogen contained in the raw steel material can be fixed by adding aluminium and carbon can be reduced by a decarbonising anneal for suppressing stretcher with yield point and for preventing the occurrence of stretcher strain.

4

Further, an increase in amount of silicon within the region of Cr≥1/3 (Al+Si) does not affect performance of the raw steel material. This means that a troublesome strict control for reducing the amount of silicon can be eliminated.

In the exemplary embodiment described above the manufacture of a shadow mask is discussed. However, the discussion is also applicable to a mask frame and an inner shield.

While the invention has been described in connection with what is presently considered to be the most practical and preferred embodiments, it is to be understood that the invention is not to be limited to the disclosed embodiments but, on the contrary, is intended to cover various modifications and equivalent arrangements included within the scope of the appended claims.

**Claims**

1. A metal component of a colour cathode ray tube, said component being formed mainly of iron and having an oxidised layer on its surface, characterised in that the metal includes silicon (Si), aluminium (Al) and chromium (Cr), the proportions by weight of these elements satisfy the equation:—

$$Cr \geq 1/3 \ (Al+Si),$$

and the combined amount of Al, Si and Cr being equal to or less than 0.44% by weight, thereby improving the adhesion of the oxidised layer to said component.

2. A metal component as claimed in claim 1, characterised in that the proportions by weight satisfy the equation:—

$$Cr \geq (Al+Si).$$

3. A metal component as claimed in claim 1 or 2, characterised in that the component is in the form of a shadow mask (14).

4. A metal component as claimed in claim 1 or 2, characterised in that the component is in the form of a mask frame (16).

5. A metal component as claimed in claim 1 or 2, characterised in that the component is in the form of a shield (24) for attachment to the mask frame of a colour cathode ray tube.

6. A colour cathode ray tube, characterised in that it includes at least one component as claimed in any preceding claim.

**Patentansprüche**

1. Metall-Bestandteil einer Farbbildröhre, welcher im wesentlichen aus Eisen gebildet ist und auf seiner Oberfläche eine oxydierte Schicht aufweist, dadurch gekennzeichnet, daß das Metall Silicium (Si), Aluminium (Al) und Chrom (Cr) enthält, wobei die Gewichtsverhältnisse dieser Elemente die Gleichung erfüllen:

$$Cr \geq 1/3 \ (Al+Si),$$

und daß die zusammengefaßte Menge von Al, Si und Cr gleich oder kleiner als 0,44 Gewichtsprozent ist, wodurch die Adhäsion der oxydierten Schicht an dem Bestandteil verbessert wird.

2. Metall-Bestandteil nach Anspruch 1, dadurch gekennzeichnet, daß die Gewichtsverhältnisse die Gleichung erfüllen:

$$Cr \geq (Al+Si).$$

3. Metall-Bestandteil nach Anspruch 1 oder 2, dadurch gekennzeichnet, daß der Bestandteil eine Schattenmaske (14) ist.

4. Metall-Bestandteil nach Anspruch 1 oder 2, dadurch gekennzeichnet, daß der Bestandteil ein Maskenrahmen (16) ist.

5. Metall-Bestandteil nach Anspruch 1 oder 2, dadurch gekennzeichnet, daß der Bestandteil ein Schild (24) zur Anbringung an dem Maskenrahmen einer Farbbildröhre ist.

6. Farbbildröhre, dadurch gekennzeichnet, daß sie zumindest einen Bestandteil gemäß einem der vorhergehenden Ansprüche aufweist.

**Revendications**

1. Composant métallique pour un tube à rayons cathodiques en couleurs, composant qui est formé principalement de fer et comporte, à sa surface une couche d'oxyde, caractérisé en ce que le métal inclut du silicium (Si), de l'aluminium (Al) et du chrome (Cr) dans des proportions en poids de ces éléments telles que l'équation suivante:

**0 079 738**

$$Cr \geq 1/3 \ (Al+Si)$$

est satisfaite et que la somme des poids de Al, Si et Cr est égale ou inférieure à 0,44% en poids, ce qui a pour effet d'améliorer l'adhérence de la couche d'oxyde audit composant.

2. Composant métallique selon la revendication 1, caractérisé en ce que les proportions, en poids, satisfont l'équation suivante:

$$Cr \geq (Al+Si)$$

3. Composant métallique selon la revendication 1 ou 2, caractérisé en ce qu'il présente la forme d'un masque perforé (14).

4. Composant métallique selon la revendication 1 ou 2, caractérisé en ce qu'il présente la forme d'un cadre de masque (16).

5. Composant métallique selon la revendication 1 ou 2, caractérisé en ce qu'il se présente sous la forme d'un écran ou d'un blindage (24) se fixant au cadre du masque d'un tube à rayons cathodiques en couleurs.

6. Tube à rayons cathodiques en couleurs caractérisé en ce qu'il contient, au moins, un composant tel que spécifié dans l'une quelconque des revendications précédentes.

6

*FIG. 1.*

A: APPLYING PHOTORESIST

↓

B: EXPOSING

↓

C: DEVELOPING

↓

D: BURNING

↓

E: ETCHING

↓

F: DECARBONIZING ANNEAL

↓

G: LEVELING

↓

H: PRESS FORMING

↓

I: DEGREASING

↓

J: OXIDIZING

FIG. 2.

FIG 3.

FIG 4.

**0 079 738**

FIG.5.

FIG.6.

FIG.7.